(19) Europäisches Patentamt
European Patent Office
Office européen des brevets

(11) Publication number: **0 338 291 A1**

# (12) EUROPEAN PATENT APPLICATION

(21) Application number: 89105459.5

(22) Date of filing: 28.03.89

(51) Int. Cl.4: A61K 31/19 , A61K 47/00

(30) Priority: 21.04.88 GB 8809424

(43) Date of publication of application: 25.10.89 Bulletin 89/43

(84) Designated Contracting States: AT BE CH DE ES FR GB GR IT LI NL SE

(71) Applicant: AMERICAN CYANAMID COMPANY
1937 West Main Street P.O. Box 60
Stamford Connecticut 06904-0060(US)

(72) Inventor: Payne, Nicholas Ian
50 Romsey Avenue Fareham
Hampshire PO16 9TA(GB)

(74) Representative: Wächtershäuser, Günter, Dr.
Tal 29
D-8000 München 2(DE)

## (54) Biphenylacetic acid gel.

(57) There is provided an antiinflammatory analgesic gel comprising a non-steroidal antiinflammatory drug of biphenyl acetic acid type, a carboxyvinyl polymer in an amount sufficient for gel formation, water as the solvent, and ethanolamine and/or L-arginine in amount sufficient to raise the pH to within the range pH 7.0 to 9.5.

EP 0 338 291 A1

# ANTIINFLAMMATORY GEL

This invention relates to a novel antiinflammatory gel containing a non-steroidal antiinflammatory drug (hereafter sometimes abbreviated to NSAID in accordance with conventional usage).

The known NSAIDS often have side-effects on the digestive organs and therefore cannot be administered to patients with peptic ulcers. To avoid this problem, it has been proposed to apply the active agent topically as a gel. However, when, as is often the case, the NSAID is insoluble or only sparingly soluble in water and other commonly used pharmaceutically acceptable solvents, it has hitherto been difficult to formulate gels in which the active agent is sufficiently solubilized in the gel in order to provide a satisfactory level of skin penetrability.

In EP-A-0127840 it is disclosed that phenylacetic acid-type antiinflammatory drugs form uniform gels of good skin penetrability with a gel base composed of a carboxyvinyl polymer as a gelling agent and a mixture of water and a lower alcohol as a solvent, if a water-soluble organic amine is incorporated in the base in an amount, in excess of that required to neutralize the carboxyvinyl polymer, sufficient to bring the pH of the final gel to pH 7.0 to 9.0. A large number of primary, secondary and tertiary water-soluble organic amines are taught as being suitable for this purpose, with diisopropanolamine being identified as being especially preferred. A gel of 4-biphenylacetic acid (also known as felbinac) formulated in accordance with the teachings of EP-A-0127840 has recently become available in some countries, and in this commercially available formulation diisopropanolamine is used as the organic amine and ethanol is used as the lower alcohol co-solvent with water.

In a specific aspect, the present invention is concerned to provide an improvement in the antiinflammatory gels disclosed in EP-A-0127840, supra.

One problem with the commercially available 4-biphenylacetic acid gel referred to above is that the presence of ethanol in the formulation limits its production to manufacturing sites which are provided with flash-proof equipment to ensure proper safety standards. On the other hand, the presence of the ethanol in the formulation does appear to enhance the absorption of the active ingredient by the skin, which often can be a matter of prime importance for ensuring the therapeutic value of the topical preparation.

Unexpectedly, we have now found that replacing the diisopropanolamine by either L-arginine or ethanolamine also permits the ethanol or other lower alcohol co-solvent to be omitted from the gel formulation without deleteriously affecting the absorption of the active ingredient by the skin. Indeed, percutaneous absorption studies with mouse skin show that the transdermal flux of 4-biphenylacetic acid from a gel containing L-arginine is about 5 times higher than a similar gel formed with diisopropanolamine, whilst a two-fold increase is observed when ethanolamine is used as the base. In contrast, a two-fold fall in the transdermal flux is observed when ethanol is omitted from the commercially available diisopropanolamine-containing gel.

These advantageous results of the use of L-arginine or ethanolamine were not at all predictable from the teachings of EP-A-0127840. Although ethanolamine is one of the many organic amines listed as being suitable in this reference, there is no suggestion that its use would permit the omission of the lower alcohol co-solvent from the gel. L-arginine is not even mentioned in EP-A-0127840, and although this naturally occurring basic amino acid has been proposed as a neutralising agent in the formation of gel bases for cosmetics in GB-A-1321024, as far as we are aware this is the first time that L-arginine has been employed in a pharmaceutical gel formulation for topical application.

Although EP-A-0127840 is concerned only with NSAIDS of phenylacetic acid type, we have found that the advantageous effects of using L-arginine or ethanolamine can be obtained also with certain other NSAIDs.

Thus, the present invention broadly provides an antiinflammatory gel comprising a non-steroidal antiinflammatory drug of biphenyl acetic acid type, a carboxyvinyl polymer in an amount sufficient for gel formation, water as the solvent, and ethanolamine and/or L-arginine in amount sufficient to raise the pH to within the range pH 7.0 to 9.5.

By the expression "non-steroidal antiinflammatory drug of biphenyl acetic acid type" as used in this specification is meant an NSAID having a structural moiety of the formula:

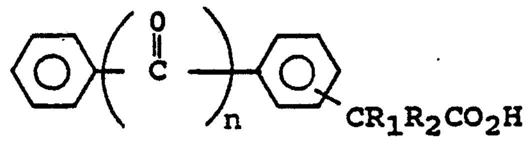

wherein n is 0 or 1; and

$R_1$ and $R_2$ are each hydrogen or alkyl.

Examples of NSAIDS of biphenyl acetic acid type useful herein include: 4-biphenylacetic acid

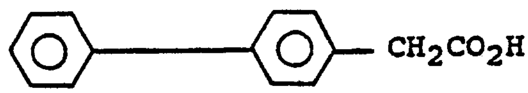

**ketoprofen**

,

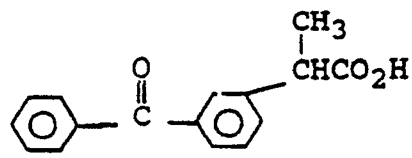

, and

**flurbiprofen**

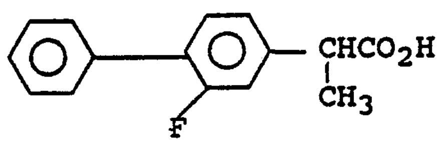

The use of 4-biphenylacetic acid (i.e. felbinac) is especially preferred.

The carboxyvinyl polymers used as a gelling agent in the gels of this invention are cross-linked acrylic acid polymers known also as carboxypolymethylenes. They are water-soluble vinyl polymers containing carboxyl groups in the molecule and having a molecular weight in the range of about 500,000 to 4,000,000. Examples of suitable carboxyvinyl polymers that can be used are Carbopol 934, 940 and 941 available from B. F. Goodrich Chemical Co., U.S.A.

The amount of the carboxyvinyl polymer in the gel is not critical, and can be varied over a wide range. Advantageously, it is from 0.5 to 5.0% by weight, preferably 0.5 to 3.0% by weight, and more preferably 0.5 to 2.0% by weight, based on the weight of the gel.

The base used in this invention is, as noted above, ethanolamine or L-arginine, or a mixture of these two amines. Ethanolamine is preferred because satisfactory gels can be formed at pH values closer to neutrality.

The amount of base used is defined by the pH of the gel, which should be within the range 7.0 to 9.5, preferably pH 8.5 to 9.2 for L-arginine-containing gels and pH 7.1 to 7.8 for ethanolamine-containing gels. Such pH values ensure that the carboxyvinyl polymer will be completely neutralized and that, preferably, there is an excess of base which will help to maintain the active ingredient in solution when, for example, subjected to temperature fluctuations during storage. Typically, the amount of the base will be within the range from 0.5 to 15.0% by weight, preferably 0.5 to 10% by weight.

As indicated above, it is an important feature of this invention that water can be used as the sole solvent and in particular that there is no need for a lower alcohol co-solvent to be present.

The gel of this invention can be prepared by conventional techniques. We prefer first to prepare a solution of the base in water, to which is then added the selected NSAID which is allowed to dissolve. Finally, the carboxyvinyl polymer is added to form the gel. No special precautions need to be taken during the preparation, unlike, for example, the ethanol-containing formulations of EP-A-0127840 which must be made in flashproof equipment with strict observance of safety standards.

The gel of this invention will normally contain a preservative such as benzyl alcohol, methyl paraben,

propyl paraben, benzalkonium chloride and thimerosal, which can be used in conventional amounts. Other optional ingredients, for example humectants such as propylene glycol and emollients, can also be incorporated, as known to those skilled in the art.

The gel of this invention may be formulated so as to have a desired viscosity, which generally will be within the range of 20,000-50,000 cps at 23 °C.

The gels of this invention can be used to treat a number of conditions, depending on the particular NSAID used, for example soft tissue injuries, extra-articular rheumatic or inflammatory conditions, acute inflammatory manifestations of osteoarthritis, sunburn, and various ocular conditions. The absence of ethanol from the formulation makes it possible to use the gels on broken skin. The gels are applied topically. Typically, the active ingredient will be present in the gel in an amount from 0.01% to 5%, preferably 1% to 3%, by weight.

The invention is illustrated by the Examples which follow.

## Example 1

A number of gels in accordance with the present invention were prepared by first adding, under stirring, either L-arginine or ethanolamine to water, whereafter the selected non-steroidal antiinflammatory drug of biphenyl acetic acid type was added until dissolved. Carbopol 940, and the optional ingredients indicated, were finally added to the solution, which then gelled.

The compositions of the resulting gels prepared in this manner are given below.

| Gel A | |
|---|---|
| Ingredient | % by weight |
| Water | 89.0 |
| L-arginine | 7.0 |
| BPAA* | 3.0 |
| Carbopol 940 | 1.0 |
| pH = 9.0 | 100.0 |

* BPAA = 4-biphenylacetic acid

| Gel B | |
|---|---|
| Ingredient | % by weight |
| Water | 84.0 |
| L-arginine | 7.0 |
| BPAA | 3.0 |
| Carbopol 940 | 1.0 |
| Propylene glycol* | 5.0 |
| pH = 9.0 | 100.0 |

*propylene glycol added as a humectant to improve "feel" of the gel

| Gel C | |
|---|---|
| Ingredient | % by weight |
| Water | 83.8 |
| L-arginine | 7.0 |
| BPAA | 3.0 |
| Carbopol 940 | 1.0 |
| Propylene glycol | 5.0 |
| Methyl parabens* | 0.2 |
| pH = 9.0 | 100.0 |

* preservative

| Gel D | |
|---|---|
| Ingredient | % by weight |
| Water | 83.72 |
| L-arginine | 7.00 |
| BPAA | 3.00 |
| Carbopol 940 | 1.00 |
| Propylene glycol | 5.00 |
| Methyl parabens | 0.25 |
| Propyl parabens* | 0.03 |
| pH 9.0 | 100.00 |

* additional preservative

| Gel E | |
|---|---|
| Ingredient | % by weight |
| Water | 93.35 |
| BPAA | 3.00 |
| Carbopol 940 | 1.00 |
| Ethanolamine | 1.65 |
| Benzyl alcohol* | 1.00 |
| pH = 7.9 | 100.00 |

* preservative

| Gel F | |
|---|---|
| Ingredient | % by weight |
| Water | 93.38 |
| BPAA | 3.00 |
| Carbopol 940 | 1.00 |
| Ethanolamine | 1.62 |
| Benzyl alcohol | 1.00 |
| pH = 7.5 | 100.00 |

| Gel G | |
|---|---|
| Ingredient | % by weight |
| Water<br>Flurbiprofen<br>Carbopol 940<br>Ethanolamine<br>Benzyl alcohol | 93.35<br>3.00<br>1.00<br>1.65<br>1.00<br>100.00 |

| Gel H | |
|---|---|
| Ingredient | % by weight |
| Water<br>Ketoprofen<br>Carbopol 940<br>Ethanolamine<br>Benzyl alcohol | 90.35<br>5.00<br>1.00<br>2.65<br>1.00<br>100.00 |

## Example 2

To test the absorbability by skin of gels of the present invention containing BPAA, the following test to determine the transdermal flux through mouse skin was conducted.

Skin excised from hairless mice was clamped between annular PTFE discs in a glass diffusion cell to provide a barrier between the donor and acceptor chambers of the cell. 10 ml of a normal saline solution (0.9% w/v NaCl), containing 0.25% w/v each of streptomycin and penicillin G to reduce bacterial growth were placed in the acceptor chamber. The test gel, in a specified amount (1.0 g) was then placed in the donor chamber of the cell, which was then closed to prevent loss by evaporation.

The diffusion cell was now stirred using a magnetic stirrer in an oven at 32° C ± 1.0° C. 0.5 ml samples were periodically extracted from the acceptor chamb er, the extracted liquid being replaced by an equal volume of 0.9% w/v saline containing streptomycin and penicillin G.

The extracted samples were stored at -20° C and analyzed by HPLC for BPAA concentration, using a Partisil 10 ODS1 column, and a mixture of 45% methanol and 55% pH 7.4 buffer (3.74% $KH_2PO_4$ and 0.77% NaOH in distilled water) as the mobile phase. Measurements were made using a wavelength of 254 nm and a flow rate of 1 ml/min.

For comparison purposes, similar transdermal flux measurements were made using the following gels:

| Comparison Gel No. 1 | |
|---|---|
| Ingredient | % by weight |
| BPAA<br>Carbopol 940<br>Ethanol (96%)<br>Diisopropanolamine (90% w/w aqueous concentrate)<br>Water<br>pH = 7.4 | 3.00<br>1.00<br>31.85<br>3.78<br>60.37<br>100.00 |

| Comparison Gel No. 2 | |
|---|---|
| Ingredient | % by weight |
| BPAA | 3.00 |
| Carbopol 940 | 1.00 |
| Diisopropanolamine (90% w/w aqueous concentrate) | 3.78 |
| Benzylalcohol | 1.00 |
| Water | 91.22 |
| pH = 7.4 | 100.00 |

The results are reported in Table I below:

TABLE I

| TRANSDERMAL FLUX OF BPAA THROUGH MOUSE SKIN - VALUES REFER TO CONCENTRATION OF BPAA AT THE RECEPTOR SIDE (µg/ml) | | | | | |
|---|---|---|---|---|---|
| TIME (HOURS) | COMPARATIVE GEL NO. 1* | ARGININE GEL OF THIS INVENTION** | COMPARATIVE GEL NO. 2 | | ETHANOLAMINE GEL OF THIS INVENTION*** |
| | Fresh Skin | Fresh Skin | Fresh Skin | Frozen Skin**** | Fresh Skin |
| 4 | 1.8 | 4.6 | - | - | - |
| 5 | 2.3 | - | - | - | 3.8 |
| 6 | - | - | 2.4 | 1.6 | - |
| 21 | 21.25 | 85.4 | 15.5 | 14.1 | 53.1 |
| 25 | - | - | 17.8 | 18.3 | - |
| 26 | 32.3 | 102.9 | - | - | - |
| 28 | 33.0 | 107.6 | - | - | - |
| 29 | 42.7 | - | 19.9 | 19.9 | 91.3 |
| 45 | 67.0 | 292.3 | 26.6 | 26.2 | 169.0 |
| 52 | 79.1 | 351.4 | 28.5 | 31.1 | 165.8 |
| 68 | 111.1 | - | - | - | 214.0 |
| 69 | 99.9 | 484.0 | - | - | - |
| 71 | - | - | 33.7 | 48.0 | - |

* composite of two separate runs
** Gel B of Example 1
*** Gel E of Example 1

From Table I it will be seen that the values obtained with Comparative Gel No. 2, of the same composition as that of Comparative Gel No. 1 except for the absence of ethanol co-solvent and the presence of benzyl alcohol, are lower at all time periods than for Comparative Gel No. 1. This is evidence to suggest that the ethanol co-solvent serves a function of enhancing the absorption of BPAA from the latter gel.

The table also shows considerably greater transdermal flux values for the two gels of this invention, with the L-arginine-containing gel promoting faster flux of drug than the ethanolamine-containing gel, despite the fact that neither gel contained any solvent other than water.

## Claims

1. An antiinflammatory gel comprising a non-steroidal antiinflammatory drug, a carboxyvinyl polymer in an amount sufficient for gel formation, water as the solvent, and ethanolamine and/or L-arginine in amount sufficient to raise the pH to within the range pH 7.0 to 9.5, wherein the non-steroidal antiinflammatory drug has a structural moiety of the formula:

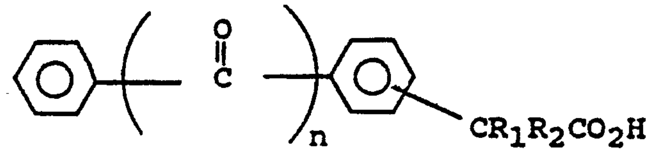

wherein n is 0 or 1, and $R_1$ and $R_2$ are each hydrogen or alkyl.

2. A gel according to Claim 1, wherein the antiinflammatory drug is 4-biphenylacetic acid.
3. A gel according to Claim 1 or Claim 2, wherein L-arginine is used.
4. A gel according to Claim 3, wherein the pH is within the range 8.5 to 9.2.
5. A gel according to Claim 1 or Claim 2, wherein ethanolamine is used.
6. A gel according to Claim 5, wherein the pH is within the range 7.1 to 7.8.
7. An antiinflammatory gel substantially as described in Example 1 or Example 2 herein.

European Patent Office

# EUROPEAN SEARCH REPORT

Application Number

EP 89 10 5459

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 105, no. 20, 17th November 1986, page 384, abstract no. 178482h, Columbus, Ohio, US; & JP-A-61 165 325 (LEDERLE (JAPAN) LTD) 26-07-1986 * Abstract * | 1-7 | A 61 K 31/19<br>A 61 K 47/00 |
| D,A | EP-A-0 127 840 (LEDERLE) * Claims * | 1-7 | |
| P,X | EP-A-0 275 054 (TOKO YAKUHIN) * Claims 1,3-6,8-10; page 2, lines 51-55; page 3, lines 1-3,6-7,14-16,30-31,48-53 * | 1-7 | |
| | | | TECHNICAL FIELDS SEARCHED (Int. Cl.4) |
| | | | A 61 K |

The present search report has been drawn up for all claims

| Place of search | Date of completion of the search | Examiner |
|---|---|---|
| THE HAGUE | 24-07-1989 | SCARPONI U. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)